# EUROPEAN PATENT APPLICATION

(11) **EP 1 913 968 A1**
(43) Date of publication of application: **23.04.2008**
(21) Application number: 07003327.9
(22) Date of filing: 16.02.2007
(51) Int. Cl.: A61M 16/04, A61M 25/02

(54) **Fixing device for endotracheal tube**

(30) Priority: 18.10.2006 KR 20060101352; 07.12.2006 KR 20060123673
(71) Applicant: Kang, Sin-Bum, Okpo 2-dong Geoje-si Gyeongsangnam-do 656-132 (KR)
(72) Inventor: Kang, Sin-Bum, Okpo 2-dong Geoje-si Gyeongsangnam-do 656-132 (KR)
(74) Representative: Klunker . Schmitt-Nilson . Hirsch

(57) **Abstract**

A device for fixing an endotracheal tube. The device includes a body (1) having an insertion hole (11), an upper wing (3) provided on a periphery of the body and a lower wing (5) provided on the periphery of the body that is spaced from the upper wing such that a patient's teeth (T) are accommodated between the lower wing and the upper wing. The device also includes a cap (9) for accommodating a portion (16a) of the body. The device fixes the endotracheal tube firmly so that it secures the endotracheal tube when surgery is being performed on a patient in a prone position. The device is movable along the teeth so that it allows surgery on the interior of the mouth or the tonsils to be performed more efficiently.

## Description

### BACKGROUND OF THE INVENTION

### FIELD OF THE INVENTION

The present invention relates to a device for fixing an endotracheal tube. More specifically, the present invention is a fixing device for an endotracheal tube having a body that has a through hole, an upper wing and a lower wing spaced apart from the upper wing so as to accommodate a patient's teeth therebetween. The present invention may further comprise a cap that can be connected to the body so as to fix an intubated endotracheal tube more firmly, to fix the endotracheal tube sufficiently during a surgical operation when the patient's position makes it difficult to fix the endotracheal tube, such as a prone position, and to offer more convenience when a dentist or a plastic surgeon performs surgery on the interior of the mouth of the patient or an otorhinolaryngologist performs surgery on the patient's tonsils, by making it possible easily to move, disconnect, or reconnect the endotracheal tube.

### DESCRIPTION OF THE RELATED ART

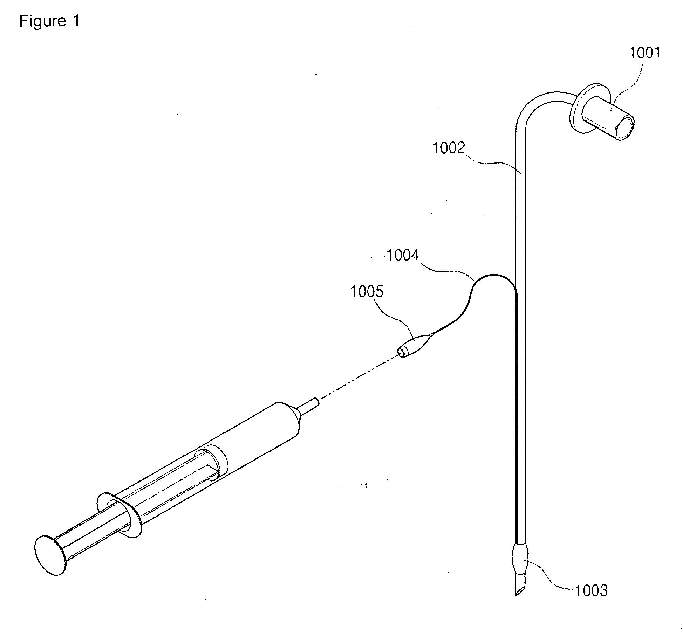

FIG. 1 shows an endotracheal tube used for securing an airway. The endotracheal tube comprises an adaptor 1001 for providing oxygen, a tubular member 1002, a cuff 1003, and a valve 1005 for inflating the cuff 1003. Those elements 1001, 1002, 1003 and 1005 may be different in size. The cuff 1003 may or may not be provided, as the occasion demands. When the cuff 1003 is not provided, it is necessary to have another means for fixing the endotracheal tube.

The adaptor 1001 is fixed to an exact position inside the airway of the patient and connected to a source of oxygen and an anesthetic to be provided to the patient. The tubular member 1002 is made from materials that are harmless to the human body, such as clear PVC, siliconized PVC or clear polyurethane. The tubular member 1002 functions as a tube by enabling the oxygen and the anesthetic to reach the patient's lungs. The cuff 1003 fixes the endotracheal tube, and prevents the oxygen or the anesthetic that is injected through the endotracheal tube from flowing backward from the lungs. The cuff 1003 also should be made from a material harmless to the human body like the tubular member 1002, and should have such a structure as to minimize the pressure applied to the wall of the patient's airway. However, it is difficult to fix the endotracheal tube firmly at an exact position by means only of the cuff 1003. The cuff-inflating valve 1005 is connected to a syringe that inflates the cuff 1003 by providing air via the cuff-inflating valve 1005 to the cuff 1003, so as to fix the endotracheal tube.

FIG. 2 describes the endotracheal tube intubated into an airway 2003 of the patient. After intubating the endotracheal tube at the desired position, the endotracheal tube can be fixed by inflating the cuff 1003 with air provided by the syringe through the cuff-inflating valve 1005 connected to the syringe. When intubating the endotracheal tube, great caution should be taken so as not to intubate the endotracheal tube into the food passage 2001. FIGs. 3 and 4 depict the cuff 1003 respectively before and after being inflated.

Tube devices such as the endotracheal tube, which are used in various medical situations, mainly aim to provide an unblocked tube to the airway of the patient who needs oxygen, medications or treatments. In most situations including especially an emergency and one requiring an intubation of the endotracheal tube for performing anesthesia, it is a matter of utmost importance to maintain the airway appropriately. Unless a suitable ventilating device or a fixing device thereof is provided, brain damage or even death may result. In addition, anesthesia is a necessary process in performing surgery because it maintains the body of the patient in the best state for the surgery, by using various anesthetics that make the patient unconscious, block the patient's senses, prevent the patient from moving, and prevent a reflex action by the patient.

The endotracheal tube to which this invention relates is one of the indispensable devices for performing general anesthesia, especially for inhalation anesthesia. General anesthesia is a kind of anesthesia in which the entire body is anesthetized by injecting a general anesthetic through the airway so as to make the patient lose his or her senses when the concentration of the anesthetic reaches a certain level. Since maintenance of the airway is very important in performing such general anesthesia, an endotracheal tube, in most cases, is intubulated into the airway before performing artificial breathing using a respirator or the hands. Accordingly, the endotracheal tube must be provided before general anesthesia and the condition of the endotracheal tube also should be checked. Moreover, the range of medical situations requiring the endotracheal tube is so broad that it is no exaggeration to say that the endotracheal tube is used in all operations that require general anesthesia. For example, the endotracheal tube is used in the following situations: when supplementing ventilation in case of insufficiency of lung ventilation; when protecting the lungs in case of an inappropriate laryngeal reflex; when treating a patient who has a high risk of inhaling contents of the stomach; when performing surgery requiring positive pressure ventilation such as a chest operation; when performing surgery while a patient is seated, lying down on one side, or in a posture of Trendelenburg where it is difficult to maintain the airway by using a mask and provide the positive pressure ventilation; when performing surgery on the cervical region or the airway, in which maintenance of the airway is required; when an anesthetist is bound to be beyond the field of view of the surgery; and when performing surgery for a patient whose airway is hard to maintain.

Such an endotracheal tube is more advantageous than other artificial respirators in the following respects: it can prevent suction of foreign substances into the airway; it can provide efficient ventilation by minimizing a dead space where no gas exchange occurs during artificial ventilation, as well as by increasing the expandability of the lungs; it can be intubulated directly into the airway without bringing about an abdominal inflation; and it makes the long-time maintenance of the airway more favorable because there is no need that it be held fast to the patient's face.

A disposable endotracheal tube made from polyvinyl chloride is the most commonly used one, since at the patient's body temperature it is deformable according to the shape of the airway and its smooth inner surface enables the disposable tube to pass through a suction tube or a bent neck of a bronchial tube. The size of the endotracheal tube is measured by its internal diameter (ID) in incrementals of five millimeters. However, the size of some tubes is measures in French size in which a number for indicating the size is determined by multiplication of the outer diameter (OD) of the endotracheal tube by π. Accordingly, endotracheal tubes with the same French size may have different inner diameters if the widths of the walls of the endotracheal tubes are different. A tube with an air bag, which is used in treating adults, or children who have reached a certain age, inflates the air bag so as to maintain a closed circuit, adjust the ventilation of the lungs, and reduce the possibility of suction of vomit or blood. A tube without the air bag, which is used in treating those who are younger than 8 years old, uses clinically the leakage pressure of the endotracheal tube to prevent the ischemia of the airway. The appropriate size of the endotracheal tube is determined by using a formula, i.e., ID = (16 + age) × ¼, or by the thickness of the fifth finger or the size of the nostrils of the small child. The appropriate sizes for grown-up men, grown-up women and children are respectively ID. 7.5~8.5 Fr, ID. 7.0~7.5 Fr, and ID. 4.0~5.5 Fr.

Now, the conventional method of intubating and fixing the endotracheal tube and its problems will be discussed.

First of all, an endotracheal tube should be selected. An appropriate endotracheal tube should be selected by taking the aforementioned various factors into consideration. Then, the doctor wearing gloves, a mask and a gown, should maintain the airway of the patient appropriately by using his or her hands while maintaining the patient's head in a neutral or a slightly dropped position, but not in an overly-dropped position. Then, a suction device for sterilization should be provided, and the blade, the body and the light bulb of a laryngoscope should be checked. The cuff of the endotracheal tube should also be checked for leakage. A fixing device, a bite block, a syringe, a stylet, a lubricant, a stethoscope, an inspirator and a tip should be provided, and finally the patient should be ventilated with the aid of an assistant.

After the above preparations have been completed, the selected endotracheal tube is ready to be intubated. The head and neck of the patient should be placed in a slightly dropped position. In so doing, great care is taken in order not to move the cervical vertebrae in the case of a patient having an external injury. Next, the doctor should push the tongue of the patient to the left by moving the laryngoscope from the left to the right side of the mouth, while grasping the laryngoscope in his or her left hand. If there is much secretion into the mouth, suction is required.

The laryngoscope is a device for viewing the glottis directly and comprises a handle, a blade and a light source. Laryngoscopes are divided into two groups based upon the type of the blade they have, i.e., a straight blade or a curved blade. A laryngoscope with an appropriately shaped blade, an appropriate light source and suitable bendability, should be provided because the airway of each person has a different anatomical shape, which is initially determined by congenital factors and changeable by acquired diseases. After inserting the blade of the laryngoscope, the doctor lifts the laryngoscope up to the extent that the lower jaw of the patient is lifted up. At this moment, the doctor should be careful to prevent damage to the patient's teeth and soft tissue, which may occur if he or she were to use the blade of the laryngoscope like a lever.

Next, the doctor intubates the endotracheal tube while keeping his eyes on the exposed glottis. During the intubation, it is better for the assistant to perform the Selick maneuver by holding and pushing both sides of the cartilage of the patient with the assistant's thumb, index finger, and middle finger until the endotracheal tube is completely intubated.

When intubating the endotracheal tube, it is necessary to make sure that the endotracheal tube is intubated into the glottis. The endotracheal tube is intubated 1~2 cm more after its distal end passes through the glottis. At the same time, the stylet is removed. The ideal depth of the intubation of the endotracheal tube is achieved when its distal end is located in the middle of the glottis and the carina. It is also noted that intubation should be performed as safely as possible to prevent infection, and that intubation should not be done in a hurry, as to a patient who is breathing on his own, since a roughly performed intubation may bring about a convulsive attack in the larynx.

After finishing the intubation, the cuff is inflated until it meets resistance, and then the syringe is removed. The cuff is inflated right after intubation is performed so as to prevent the aforementioned suction into the airway. However, overexpansion of the cuff should be avoided because it may bring about damage to the mucous membrane, the airway, and the gullet.

Next, the depth of the intubation is measured by the scale markings on the side surface of the endotracheal tube. The depth should be changed if necessary after checking whether there is appropriate intubation by auscultating the breathing sound in both lungs and the stomach. The appropriate intubation depth can be checked by determining whether the breathing sounds of both lungs are equal, whether a stomach sound is audible during ventilation, whether there is moisture on the surface of the tube during ventilation, whether the patient's chest has expanded enough, and whether the endotracheal tube is confirmed to have passed through the glottis in case of intubation through the mouth.

Finally, the endotracheal tube should be continuously held by hands so as to maintain its position. A bite block, which is inserted after intubation, prevents the patient from suffocating. In addition, in the case of a patient who is unable to breath on his own, the time during which the artificial breathing is stopped for intubation of the endotracheal tube should be less than 30 seconds.

While the endotracheal tube has many merits, it can cause a complication after the surgery has been completed, such as a sore throat and hoarseness. The sore throat and hoarseness may occur when general anesthesia has been performed through the endotracheal tube intubated into the airway. Even though the sore throat is a temporary complication from which the patient can recover naturally within 72 hours, it can bring about serious discomfort to the patient. The frequencies of a sore throat and the hoarseness after the surgery are known to be between 24 to 65% and between 13 to 40% respectively. The cause of the sore throat and hoarseness may be, for example, the diameter of the endotracheal tube, the type of the cuff, or the use of a lubricant.

An overexpansion of the cuff is known to be the main reason for such complications. Conventionally, lidocaine has been used to eliminate the sore throat. For example, lidocaine is injected into the cuff and spreads through the membrane of the cuff so as to anesthetize the mucous membrane of the airway. However, recent research shows that lidocaine is not so effective. Rather, an appropriate expansion of the cuff would be the best way to eliminate the sore throat. To inflate the cuff appropriately, it is important to fix the endotracheal tube taking into account that the cuff is an element for fixing the endotracheal tube. However, in conventional devices, the fixing of the cuff has been achieved only by the cuff itself, which inevitably has brought about an overexpansion of the cuff, that is, an expansion beyond the minimum range of expansion needed to prevent the oxygen or anesthetic from flowing backward.

In practice, it is very hard to select an appropriate endotracheal tube and to intubate and fix it. Even an experienced doctor frequently makes a mistake, which may resulting from any of many factors, such as the physical diversity of patients and the diversity of situations where the airway should be fixed. Namely, since the doctor intubates the endotracheal tube into the airway of the patient depending only upon his eyesight to guide him, it is possible mistakenly to intubate the endotracheal tube inside the gullet.

In addition, over-intubation may force the endotracheal tube to be intubated into a blood vessel that is connected to only a single lung of the patient, so that it provides air to one lung rather than to both lungs. In such a case, the result may be fatal. Such a result may also occur if the patient consciously or unconsciously touches the endotracheal tube which has been intubated correctly. As a result, it is true that a variety of fatal medical accidents can occur when the endotracheal tube is not fixed firmly enough.

Further, even when the endotracheal tube is intubated correctly, a number of different difficulties may follow in fixing the intubated tube. Conventionally, the endotracheal tube has been fixed by wrapping an adhesive bandage around the endotracheal tube and by applying the bandage to the skin around the mouth. However, such a method does not fix the intubated endotracheal tube strongly enough as reliably to prevent it from moving. It is also possible for the patient consciously or unconsciously to pull out the endotracheal tube with his hand. Especially, an endotracheal tube intubated into the airway of a young child or an infant may deviate from the correct position in the airway even with a small movement of the endotracheal tube. Further, the adhesive bandage may bring about a contact dermatitis to a patient with sensitive skin. Such examples have been clinically reported many times.

Moreover, generally two persons are required to fix the endotracheal tube by using the adhesive bandage, wasting manpower. It is also possible for the endotracheal tube to move when the patient moves his neck due to pain felt when removing a secretion such as phlegm in the airway, or when his saliva causes the bandage to become detached. These problems are most serious in a young child or an infant.

To solve these problems, it has been taken into consideration to fix the endotracheal tube by binding the endotracheal tube to the neck of the patient with a band that is attached to the endotracheal tube. However, even with this technique, it is still very difficult to fix the endotracheal tube perfectly in the exact position. Even when the tube is exactly fixed at the first stage, it is still necessary to move the fixed endotracheal tube, as the occasion demands. In such a case the doctor should remove the band, relocate the endotracheal tube and bind the band again, which is burdensome in a medical emergency. In addition, the bite block prevents the patient from biting the endotracheal tube and, accordingly, from suffocating. The bite block also functions as an airway for ventilation. Conventionally, when suction is required to remove foreign substances in the mouth or airway, the suction has been performed using a space that remains after inserting the bite block. However, in the conventional way of fixing the endotracheal tube by using a band or the like, such suction cannot be performed efficiently because of the difficulty in securing enough space for performing suction or the difficulty in moving the suction device.

Further, when performing a surgical operation in the interior of the mouth or on a patient in a prone position, the conventional method of binding a band is not sufficient for performing the surgery because it makes movement of the fixed endotracheal tube more probable. Thus, the conventional method has provided medical services that are neither fast nor proper. The problem increases in the case of surgery for a patient in a prone position, where it is impossible to fix the intubated endotracheal tube completely by the conventional band binding method. Thus, the possibility still exists that permanent damage including death could occur. Moreover, the cuff is more likely to be overinflated in the conventional method, resulting in many complications after the intubation of the endotracheal tube, such as a sore throat.

Considering that the endotracheal tube is usually required in a very urgent emergency, the conventional method of fixing the endotracheal tube by binding a band is very problematic both in the speed and the reliability of the fixation. When a dentist or a plastic surgeon performs surgery on the interior of the mouth, or an otorhinolaryngologist performs surgery on the tonsils, the procedure for moving the fixed endotracheal tube in the conventional method is very burdensome and difficult, as aforementioned. Likewise, in order to remove the foreign substance, such as phlegm, from the mouth or the airway, the conventional method also requires the fixed band to be detached, the endotracheal tube to be intubated again, and the intubated endotracheal tube again to be fixed.

### SUMMARY OF THE INVENTION

To solve the foregoing problems, it is an object of the present invention to provide a device for fixing an endotracheal tube that can fix the endotracheal tube faster and more firmly when intubation of the endotracheal tube is required in an emergency or for putting a patient under anesthesia.

It is another object of the present invention to provide a device for fixing an endotracheal tube that enables a single person to fix the endotracheal tube easily and exactly.

It is another object of the present invention to provide an endotracheal tube fixing device comprising a multipurpose hole, which provides tighter fixation as well as simpler and easier suction when such suction is necessary.

It is another object of the present invention to provide an endotracheal tube fixing device comprising a lower wing that can be modified according to the situation in which the endotracheal tube is used, or the usage to which the endotracheal tube is put, so as to meet various kinds of emergency.

It is another object of the present invention to provide an endotracheal tube fixing device, which is movable along a patient's teeth, as the occasion demands, after fixing the endotracheal tube for surgery.

It is another object of the present invention to provide an endotracheal tube fixing device, which provides a safe and reliable fixation of the endotracheal tube and can continuously maintain the endotracheal tube exactly at the fixed position when the tube is used in surgery with a high possibility for the intubated endotracheal tube to deviate from the initially fixed position, such as surgery requiring that a patient be in a prone position.

It is another object of the present invention to provide an endotracheal tube fixing device comprising an upper wing that is so movable as to meet various mouth structures and teeth structures of patients, and as to make it possible to maintain an airway without use of an additional device.

It is another object of the present invention to provide an endotracheal tube fixing device, which fixes the endotracheal tube accurately and safely, so as to minimize overexpansion of a cuff and so as to prevent a complication such as a sore throat as may result from such overexpansion.

To achieve the above objects, the present invention may be realized by any of the following exemplary embodiments.

According to an embodiment of the present invention, there is provided an endotracheal tube fixing device that includes a body having an insertion hole, an upper wing provided on a periphery of the body, and a lower wing provided on a periphery of the body, the lower wing being spaced apart from the upper wing such that the patient's teeth are accommodated between the lower wing and the upper wing.

According to another embodiment of the present invention, the device further includes a cap that has a concave portion for accommodating a portion of the body so as to fix the endotracheal tube after the endotracheal tube is inserted into the insertion hole.

According to another embodiment of the present invention, the device further includes an insertion element that is inserted into the insertion hole after the endotracheal tube is inserted into the insertion hole.

According to another embodiment of the present invention, the device further includes a bonding member that bonds the body and the endotracheal tube together after the endotracheal tube is inserted into the insertion hole.

According to another embodiment of the present invention, the body of the device includes a first body and a second body and the first body and the second body are connected together so as to fix the endotracheal tube after the endotracheal tube is inserted into the insertion hole.

According to another embodiment of the present invention, the first body includes a push-fitting boss and the second body comprises a push-fitting groove.

According to another embodiment of the present invention, the push-fitting boss is larger than the push-fitting groove.

According to another embodiment of the present invention, the first body includes a concave portion and the second body comprises a convex portion.

According to another embodiment of the present invention, the first body includes a male latch element and the second body comprises a female latch element.

According to another embodiment of the present invention, the body includes a first portion protruding from the first wing and the first portion has a cut groove.

According to another embodiment of the present invention, the cap has an inclined sidewall so that the radius of the cap at its lower portion is bigger than at its higher portion.

According to another embodiment of the present invention, the cap has a thread on an inner surface of its sidewall and the body has a thread on its outer surface.

According to another embodiment of the present invention, the inner surface of the sidewall is inclined so that the radius of the cap at a lower portion of the inner surface is bigger than at a higher portion of the inner surface.

According to another embodiment of the present invention, the body includes a push-fitting groove on an outer surface thereof and the cap has a push-fitting boss corresponding the push-fitting groove on an inner surface thereof.

According to another embodiment of the present invention, the push-fitting boss is bigger than the push-fitting groove.

According to another embodiment of the present invention, the body has a concave portion on an outer surface thereof and the cap has a convex portion on an inner surface thereof.

According to another embodiment of the present invention, the body has a female latch element on an outer surface thereof and the cap has a male latch element on an inner surface thereof.

According to another embodiment of the present invention, the cap has a first part and a second part, and the first part and the second part are connected together and a portion of the body is accommodated therein so as to fix the endotracheal tube.

According to another embodiment of the present invention, the first part has a push-fitting boss and the second part has a push-fitting groove.

According to another embodiment of the present invention, the push-fitting boss is bigger than the push-fitting groove.

According to another embodiment of the present invention, the first part has a concave portion and the second part has a convex portion.

According to another embodiment of the present invention, the first part has a male latch element and the second part has a female latch element.

According to another embodiment of the present invention, the upper wing is spaced apart from the body and the body has bosses on an outer surface thereof and the upper wing is movable along the outer surface of the body so as to be usable on patients with various shapes of the teeth and the interior of the mouth.

According to another embodiment of the present invention, the body has a thread on an outer surface thereof, the upper wing has a thread on an inner surface thereof, and the upper wing is slidable and fixable along the outer surface of the body.

According to another embodiment of the present invention, the device further comprises an airway maintaining element, connected with lower wing, which keeps the airway open.

According to another embodiment of the present invention, the airway maintaining element comprises a hollow portion into which the endotracheal tube is inserted, and a wing adjacent to the side of the hollow portion.

According to another embodiment of the present invention, the upper wing has a plurality of multipurpose holes.

According to another embodiment of the present invention, the lower wing has a plurality of multipurpose holes.

According to another embodiment of the present invention, the cap includes a guide surface at a bottom thereof.

According to another embodiment of the present invention, the lower wing is balloon-shaped so that the lower wing is contracted in the course of inserting and extracting the device, and the lower wing inflates while the device fixes the endotracheal tube.

According to another embodiment of the present invention, the upper wing has a plurality of multipurpose holes.

According to another embodiment of the present invention, the upper and lower wings each have a concave portion.

According to another embodiment of the present invention, the concave portion is circular or oval.

According to another embodiment of the present invention, the body has an insertion groove for fitting therein a tube that is used to inflate the cuff.

According to another embodiment of the present invention, the insertion groove is smaller in diameter than the tube.

According to another embodiment of the present invention, the body has a cut groove.

According to another embodiment of the present invention, the cap has a sidewall whose inner surface is inclined.

According to another embodiment of the present invention, the cap is partially coupled with the body.

### BRIEF DESCRIPTION OF THE DRAWINGS

These and other aspects of the invention may better be understood from the following detailed description of preferred embodiments with reference to the accompanying drawings in which:
FIG. 1 is a perspective view showing an endotracheal tube and a syringe used for inflating a cuff.

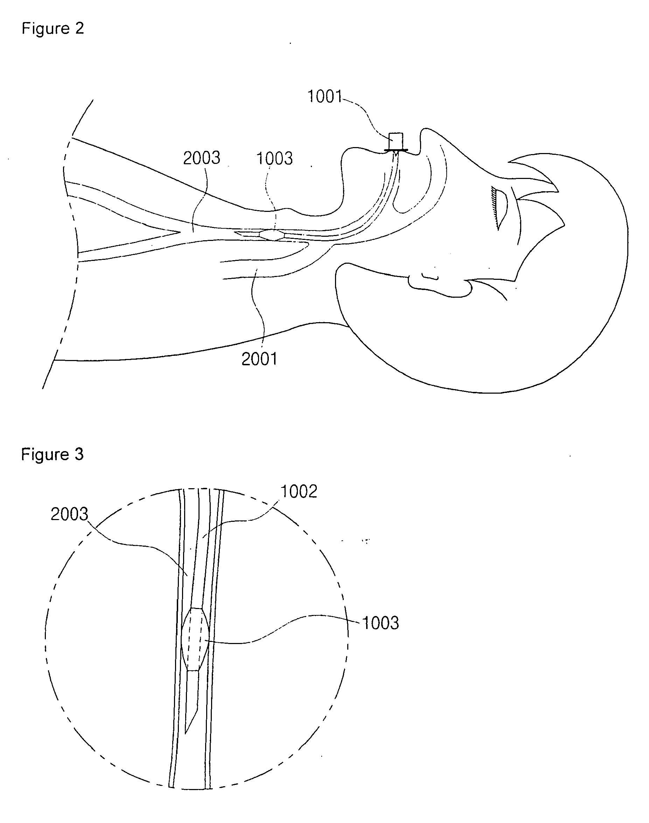

FIG. 2 is a side view showing an endotracheal tube intubated into a patient.

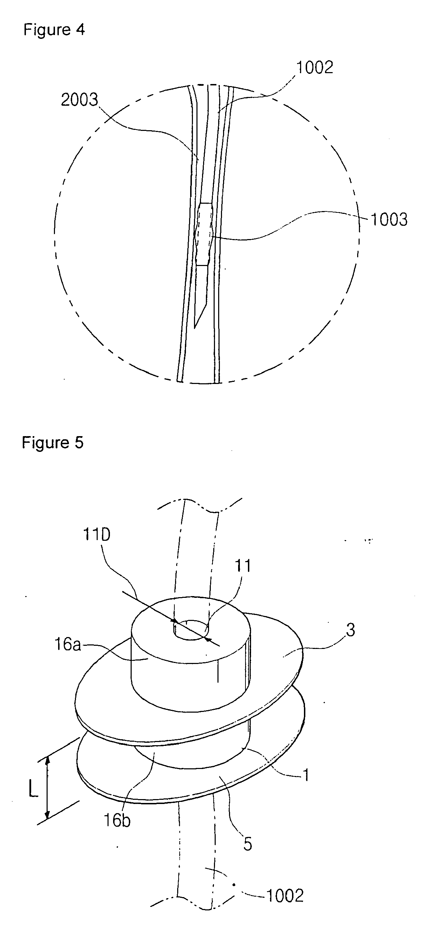

FiGs. 3 and 4 are sectional views respectively showing a cuff after expansion and a cuff before expansion.
FIG. 5 is a perspective view showing an endotracheal tube fixing device according to an embodiment of the present invention.

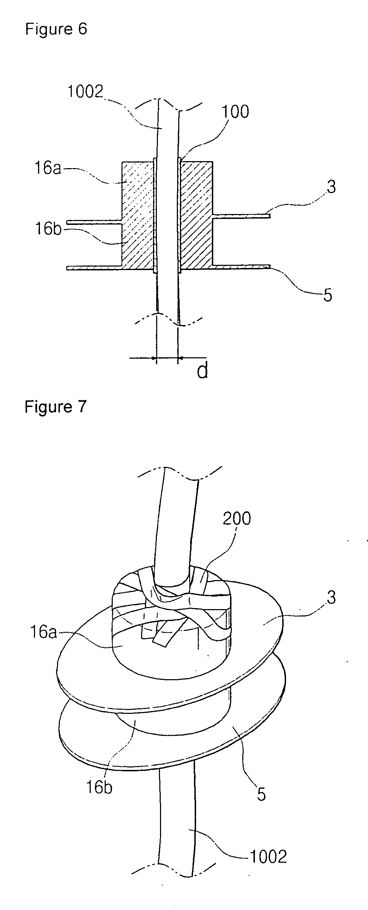

FIGs. 6 and 7 are respectively a sectional view and a perspective view showing an endotracheal tube fixing device according to another embodiment of the present invention.

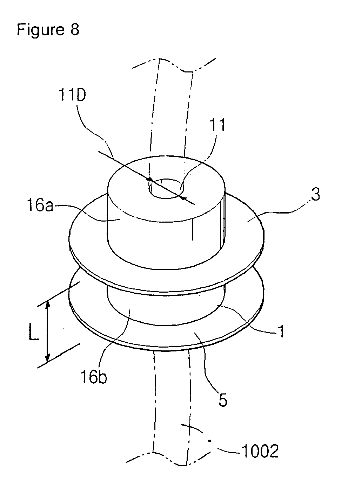

FIG. 8 is a perspective view showing an endotracheal tube fixing device with an upper wing and a lower wing according to another embodiment of the present invention.

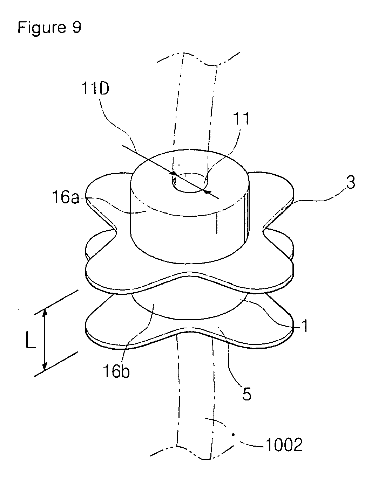

FIG. 9 is a perspective view showing an endotracheal tube fixing device with other upper and lower wings according to another embodiment of the present invention.

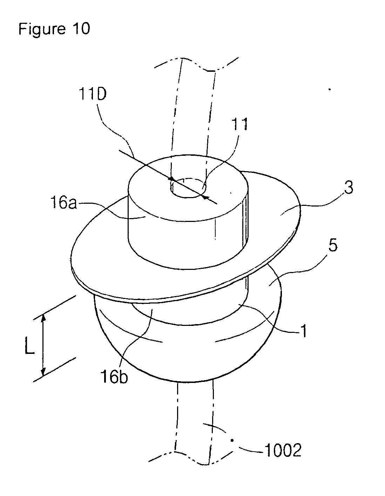

FIG. 10 is a perspective view showing an endotracheal tube fixing device having a lower wing according to another embodiment of the present invention.

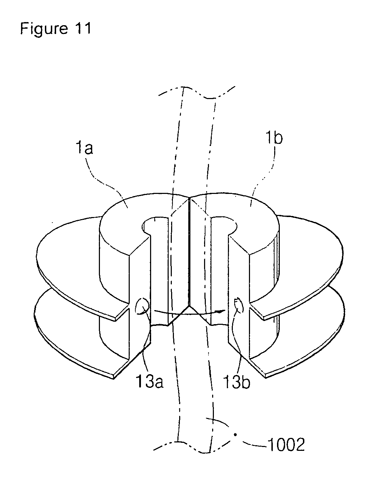

FIG. 11 is a perspective view showing an endotracheal tube fixing device according to another embodiment of the present invention.

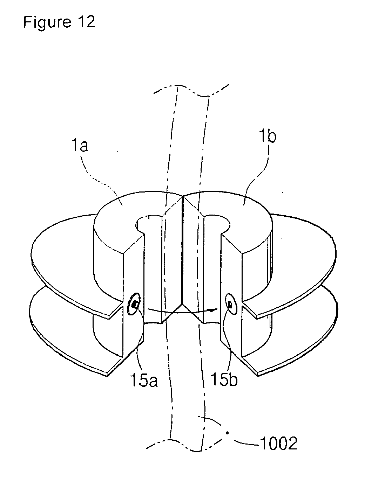

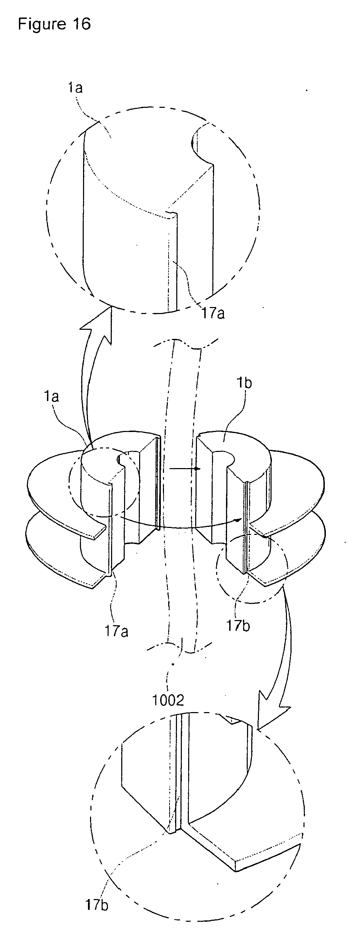

FIGs. 12 - 16 are perspective views respectively showing endotracheal tube fixing devices according to other embodiments of the present invention.

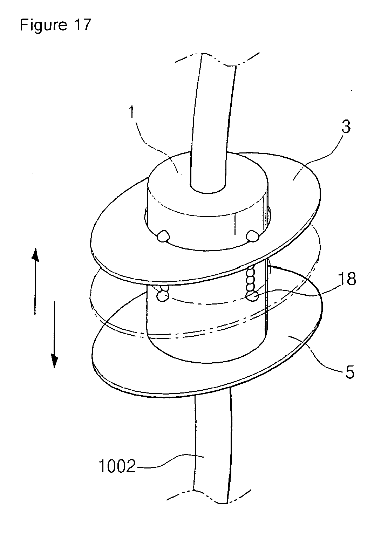

FIG. 17 is a perspective view showing an endotracheal tube fixing device having an upper wing that is movable along bosses formed thereon, according to another embodiment of the present invention.

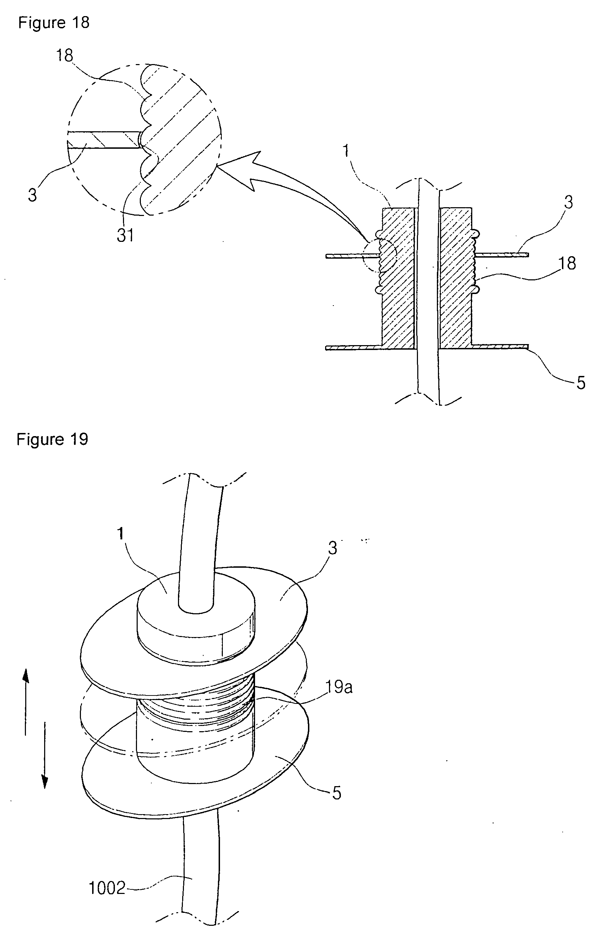

FIG. 18 is a sectional view of the endotracheal tube fixing device of FIG. 17.
FIG. 19 is a perspective view showing an endotracheal tube fixing device having an upper wing that is movable along threads formed thereon, according to another embodiment of the present invention.

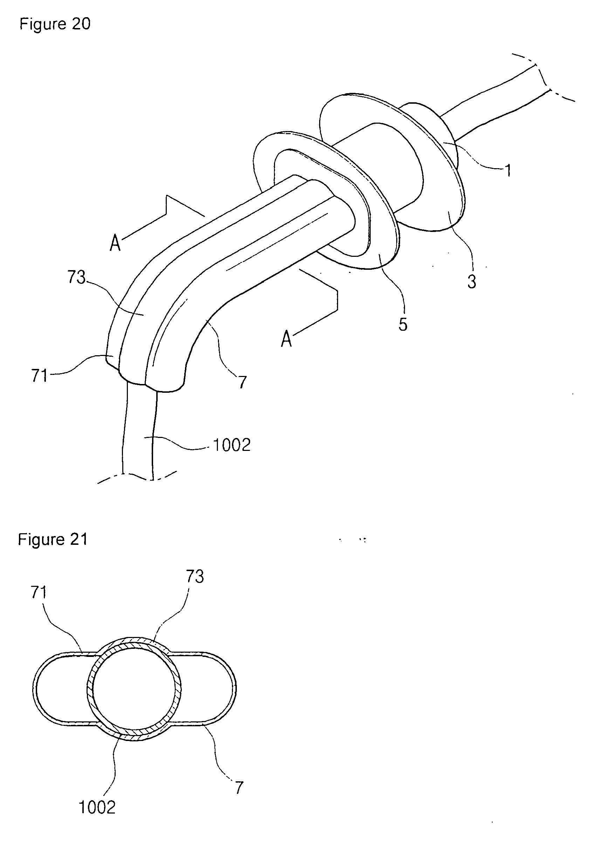

FIG. 20 is a perspective view showing an endotracheal tube fixing device having an airway maintaining element, according to another embodiment of the present invention.
FIG. 21 is a sectional view taken along the A-A line in FIG. 20, of an endotracheal tube fixing device.

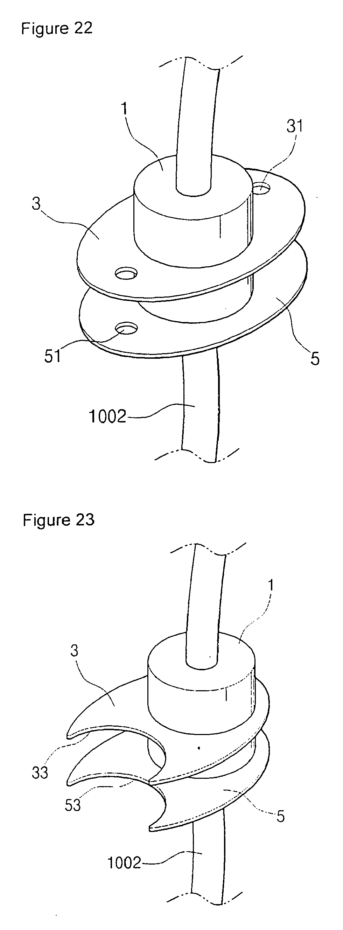

FIG. 22 is a perspective view showing an endotracheal tube fixing device having multipurpose holes in an upper wing and a lower wing thereof, according to another embodiment of the present invention.
FIG. 23 is a perspective view showing an endotracheal tube fixing device having concave portions formed in an upper wing and a lower wing thereof, according to another embodiment of the present invention.

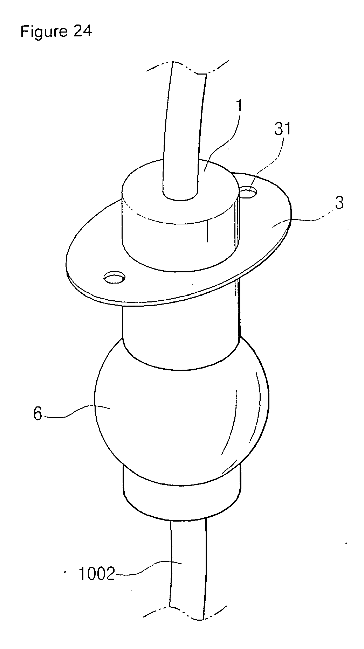

FIG. 24 is a perspective view showing an endotracheal tube fixing device having a balloon-shaped lower wing, according to another embodiment of the present invention.

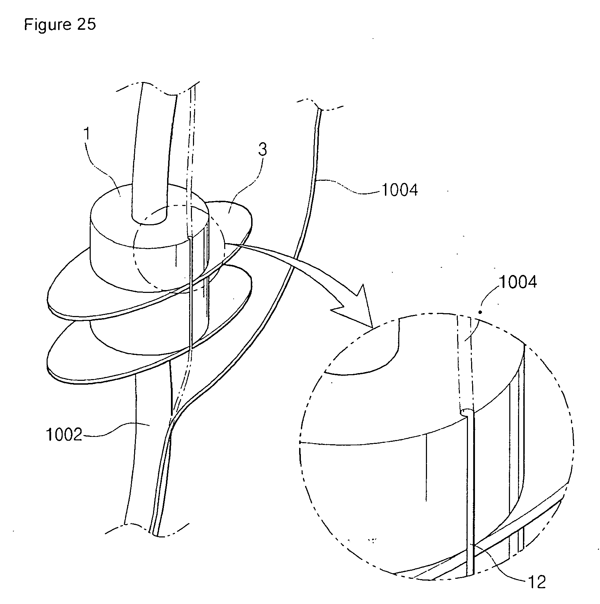

FIG. 25 is a perspective view showing an endotracheal tube fixing device having an insertion groove, according to another embodiment of the present invention.

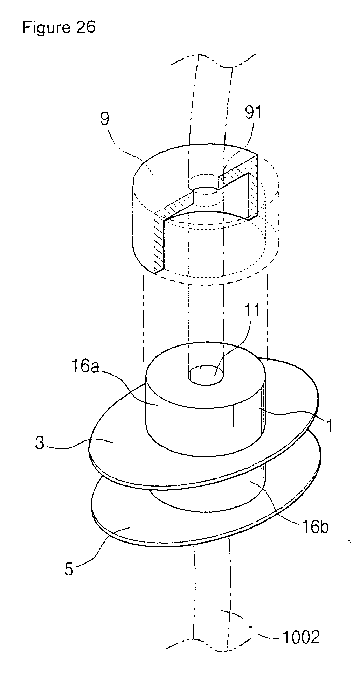

FIG. 26 is a perspective view showing an endotracheal tube fixing device having a cap, according to another embodiment of the present invention.

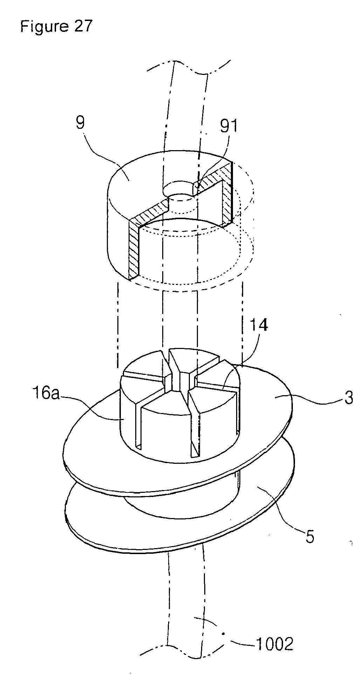

FIG. 27 is a perspective view showing an endotracheal tube fixing device having a cut groove, according to another embodiment of the present invention.

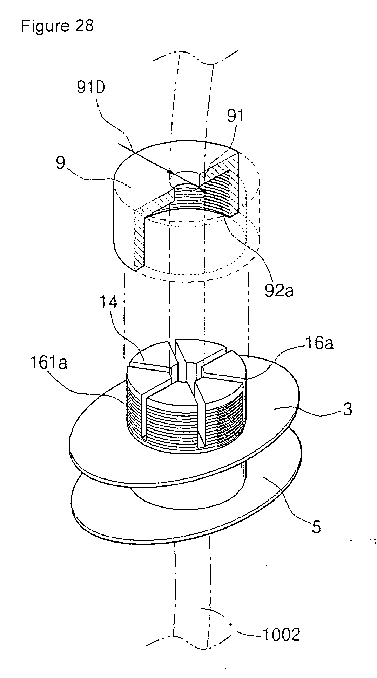

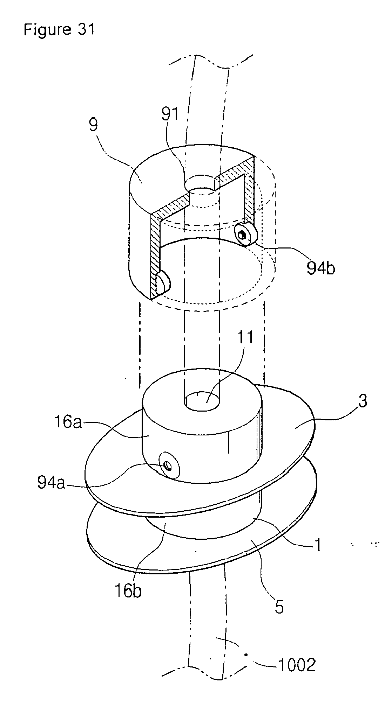

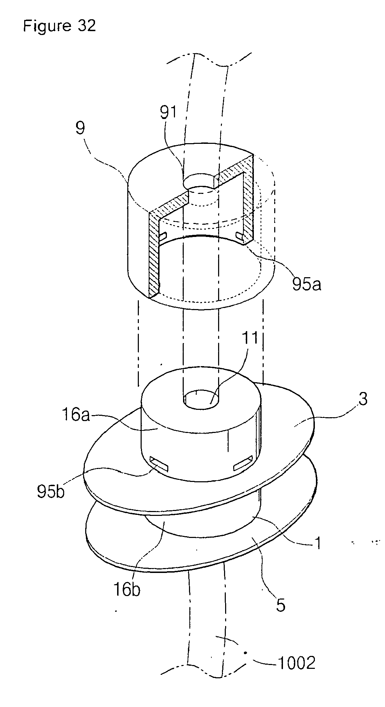

FIGs. 28 - 33 are perspective views respectively showing endotracheal tube fixing devices having caps of different shapes, according to other embodiments of the present invention.

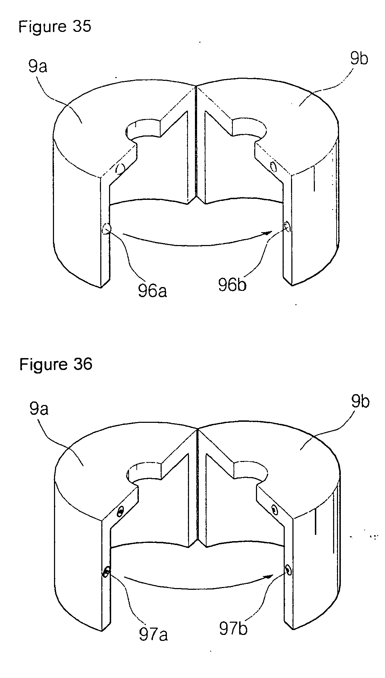

FIGs. 35 - 37 are perspective views respectively showing caps which are divided into two parts with an edge of one part being coupled with an edge of the other part while two parts can be assembled together, according to other embodiments of the present invention.

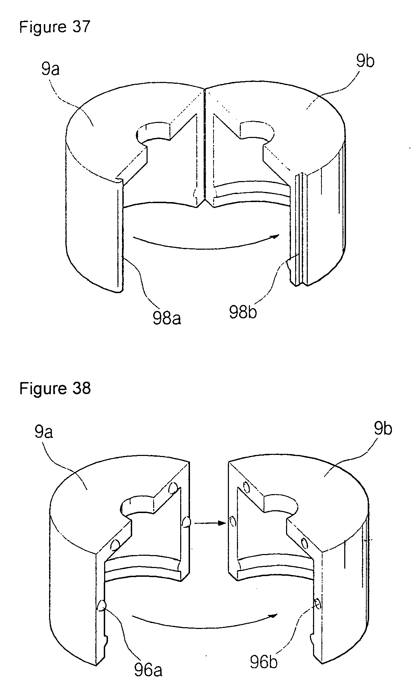

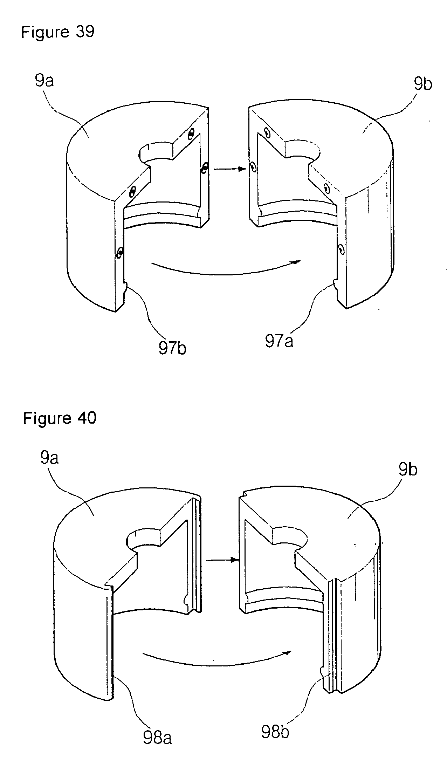

FiGs. 38 - 40 are perspective views respectively showing caps which are divided into two parts according to another embodiment of the present invention.

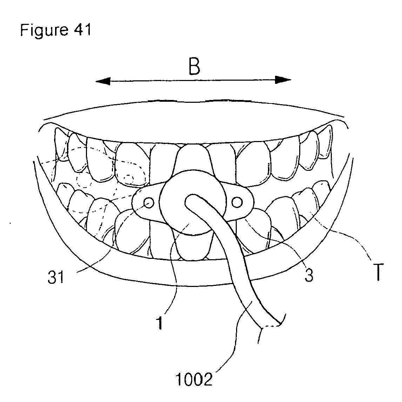

FIG. 41 is a front view showing that an endotracheal tube fixing device of the present invention is movable along the patient's teeth.

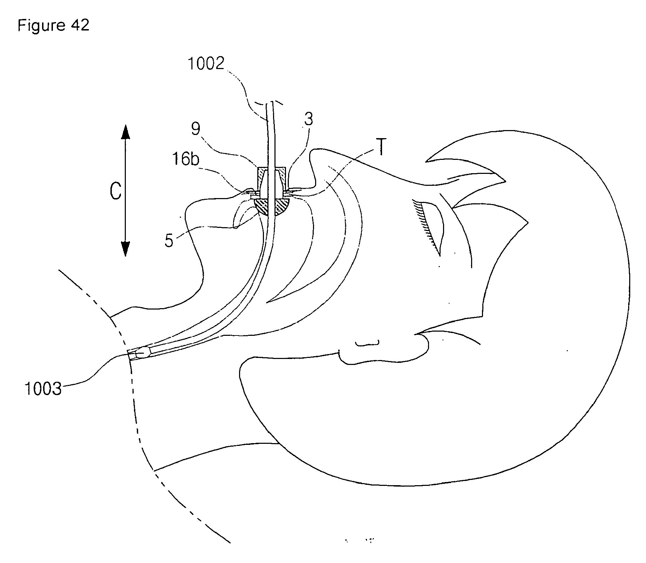

FIG. 42 is a side sectional view showing an endotracheal tube fixing device of the present invention, placed in a patient.

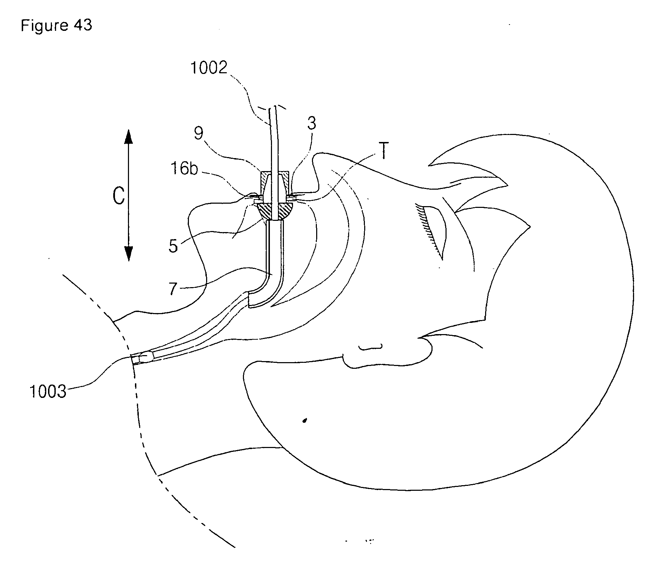

FIG. 43 is a side sectional view showing an endotracheal tube fixing device having an airway maintaining element of the present invention, placed in a patient.

### DETAILED DESCRIPTION OF THE INVENTION

Reference now will be made in detail to embodiments of the present invention which are illustrated in the accompanying drawings.

Referring to FIG. 5, an endotracheal tube fixing device according to an embodiment of the present invention comprises a body 1, and an upper wing 3 and a lower wing 5 that are generally disc-shaped and surround the body 1.

The body 1 is hollowed to define an insertion hole 11 through which the endotracheal tube is inserted, and, preferably, has shape of a hollow cylinder. The diameter 11 D of the insertion hole 11 should be determined according to the diameter d of the endotracheal tube which is inserted. The diameter 11 D will differ among various embodiments of the present invention as will be described in detail below.

The body also has a first portion 16a that protrudes from the upper wing 3 and a second portion 16b between the upper wing 3 and the lower wing 5. The first portion 16a, protruding from the upper wing 3, protrudes from the patient's teeth when the endotracheal tube has been fixed, and enables the upper wing 3 to move along the first portion 16a so as to meet various shapes of the teeth or mouth, which vary from patient to patient.

The second portion 16b between the upper and lower wings 3 and 5 is engaged with the patient's teeth when the endotracheal tube has been fixed. The length of the second portion 16b varies as the upper wing 3 moves along the body 1.

Since the second portion 16b is hollow and preferably has a shape of a hollow cylinder, and it is engaged with the patient's teeth while the endotracheal tube is being fixed, the second portion 16b is preferably made from a material with sufficient rigidity to prevent the endotracheal tube from contracting if the patient consciously or unconsciously bites the second portion 16b strongly. In this way, the wall of the endotracheal tube is prevented from blocking inhalation of air therethrough. However, it is also necessary to choose a material that does not damage the patient's teeth.

In addition, the second portion 16b preferably has such a length L that the upper wing 3 is located so as to contact the front surface of the teeth while the lower wing 5 is located so as to contact the rear surface of the teeth, thereby to prevent the fixing device from moving backward and forward, for example, in the direction C shown in FIG. 42, while allowing the fixing device to move from side to side, for example in the direction B shown in FIG. 41.

Since the patient does not bite the second portion 16b so firmly as to prevent any movement of the device for fixing the endotracheal tube, it is possible to make it easier for dentists or plastic surgeons to perform surgery on the interior of the mouth, or an otorhinolaryngologist to perform surgery on the tonsils, by moving the device of the present invention easily along the patient's teeth, as the occasion demands.

As aforementioned, conventionally suction has been performed using the space available after inserting the bite block. However, in the conventional way of fixing the endotracheal tube, such suction can not be performed efficiently because it is difficult to secure enough space for performing suction, as well as to move the endotracheal tube, as the occasion demands. The present invention solves this problem by providing a fixing device that is movable along the patient's teeth.

Referring to FIG. 6, the endotracheal tube is inserted into the insertion hole 11 by inserting a supplementary insertion member 100 when the diameter d of the endotracheal tube is smaller than the diameter 11 D of the insertion hole 11.

It is true that the diameter 11 D of the insertion hole best corresponds with the diameter d of the endotracheal tube. However, even when the diameter d is smaller than the diameter 11 D of the insertion hole 11, the supplementary insertion member 100 inserted between the endotracheal tube and the insertion hole 11 makes it possible to prevent the inserted endotracheal tube from moving.

The supplementary insertion member 100 is not limited in its form. For example, a piece of paper can be inserted as the supplementary insertion member 100 in an emergency.

Referring to FIG. 7, a bonding member 200 is used to prevent the endotracheal tube from moving when the diameter d of the endotracheal tube is smaller than the diameter 11 D of the insertion hole 11. The bonding member 200 is bound around a tubular member 1002 of the endotracheal tube and, then, is bonded to the fixing device of the present invention.

The bonding member 200 also is not limited in its form. For example, an adhesive bandage can be used.

FIGs. 8 - 10 show various embodiments of the upper wing 3 and the lower wing 5 according to other embodiments of the present invention,

The upper wing 3 faces the front side of the patient's teeth and is visible from the outside, while the lower wing 5 faces the back side of the teeth and is not visible from the outside. Both wings 3 and 5 together prevent the fixing device of the present invention from moving backward and forward, thereby prevent the intubated endotracheal tube from moving backward and forward, while allowing movement from side to side along the teeth so as to make it easy to perform cleaning or suction inside the mouth, as the occasion demands.

Referring to FIGs. 8 and 9, preferably, the upper wing 3 and the lower wing 5 are circular, oval, or propeller-shaped, while both wings 3 and 5 have a hollowed portion into which the body 1 is inserted.

An oval wing is usefully provided therein with a larger multipurpose hole, which will be discussed below. A circular wing is useful to broaden the range of the movement of the fixing device of the present invention. A propeller-shaped wing with a broader area facing the teeth is useful to fix the intubated endotracheal tube more firmly.

FIG. 10 shows another embodiment of the lower wing 5. The lower wing 5 in FIG. 10 has a smooth, curved surface so that the fixing device of the present invention can reduce the patient's rejection of the inserted fixing device and, thus, does not cause the patient to vomit. The lower wing 5 in FIG. 10 also has a hollowed portion into which the body 1 is inserted. The upper wing 3 may have the shape of the lower wing 5 in FIG. 10, although such a shape is likely to be less advantageous as the upper wing 3 than as the lower wing 5.

FIG. 11 shows a fixing device according to another embodiment of the present invention. Depicted in FIG. 11 is a fixing device having a body 1 that is divided into two parts, i.e., a first body 1a and a second body 1b. An edge of the first body 1 a is coupled with an edge of the second body 1 b.

In this embodiment, it is preferable also to divide each of the upper wing 3 and the lower wing 5 into two parts respectively coupled with the first body 1 a and the second body 1b.

The first body 1 a includes a push-fitting boss 13a for coupling the first body 1 a with the second body 1b, while the second body 1b includes a push-fitting groove for fitting the push-fitting boss 13b.

The body 1 may have any number of push-fitting bosses 13a and the push-fitting grooves 13b in any shape as would permit the push-fitting boss 13a to be fitted in the push-fitting groove 13b so as to couple the first body 1 a with the second body 1b. Given that the fixing device of the present invention may be formed as a disposable medical device, it is preferable that the push-fitting boss 13a is bigger than the push-fitting groove 13b in order to accomplish the push-fitting between the boss 13a and the groove 13b.

Referring to FIG. 12, instead of the push-fitting boss 13a and the push-fitting groove 13b, the body 1 has a concave portion 15b and a convex portion 15a. Such a method of coupling the first body 1 a and the second body 1b together uses what is generally known as a press-button type coupling, which need not be explained in detail.

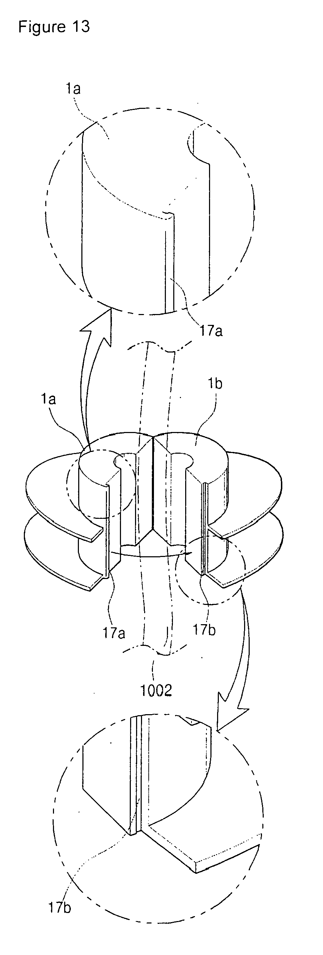

Referring to FIG. 13, instead of the push-fitting boss 13a and the push-fitting groove 13b, the body may have a male latch element 17a and a female latch element 17b. Such a method of coupling the first body 1 a and the second body 1b together uses what is generally known as a latch-type coupling. The latch-type coupling is conventionally used, for example, in coupling a battery to the main body of a cell phone. In the present invention, the male latch element 17a is inserted by force into the female latch element 17b so that they are combined together to couple the first body 1 a with the second body 1 b.

Although FIGs. 11-13 respectively show the first body 1a having the push-fitting boss 13a, the convex portion 15a and the male latch element 17a and the second body 1b having the push-fitting groove 13b, the concave portion 15b and the female latch element 17b, it is also possible to reverse the location of the respective coupling elements.

When the first and second bodies 1 a and 1b are coupled together according to the embodiments depicted in FIGs. 11-13, the insertion hole 11 is formed after coupling of the first and second bodies 1 a and 1 b. Preferably, the diameter 11 D of the insertion hole 11 is smaller than the diameter d of the endotracheal tube so that by pressing the inserted tubular member 1002 the endotracheal tube is fixed firmly without additional fixing elements. It is apparent that other fixing mechanisms in the aforementioned embodiments also are available.

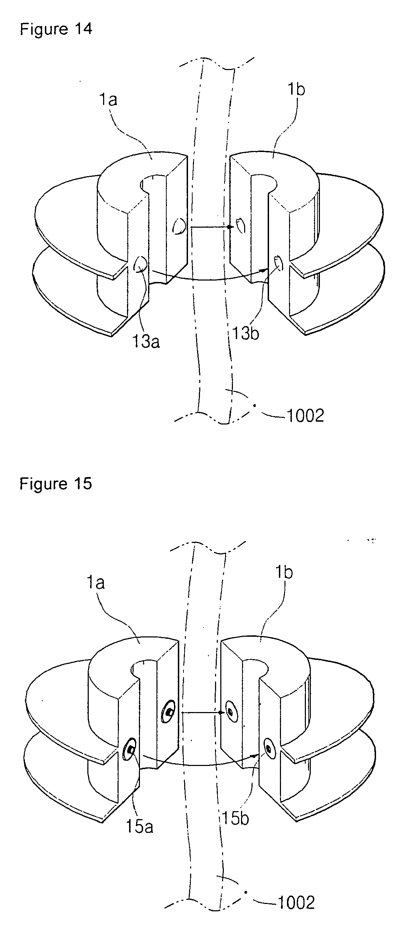

Referring to FIGs. 14-16, the first body 1a and the second body 1b are completely separated into two parts and, then, the two parts are coupled together by using the aforementioned embodiments so as to fix the intubated endotracheal tube. The push-fitting boss 13a, the concaved portion 15a and the male latch elements 17a can be adopted in the same way as aforementioned, and so the details are omitted.

Referring to FIG. 17, the upper wing 3 is movable along the outer surface of the body 1. The outer surface of the body 1 has bosses 18 thereon and the upper wing 3 is connected to the body while a space is left between the body 1 and the upper wing 3.

The reason for providing such an upper wing 3 movable along the body 1 is that each patient has a different shape of the teeth and interior of the mouth. If the upper wing 3 is excessively spaced from the lower wing 5, it is difficult to fix the intubated endotracheal tube. Likewise, it is also problematic when the distance between the upper and lower wings 3 and 5 is so short.that the patient's teeth cannot be inserted between the two wings. Accordingly, by providing such movability to the upper wing 3, the fixing device of the present invention enables the upper and lower wings 3 and 5 to contact the teeth when the second portion 16b of the body 1 is engaged with the teeth so that it can fix the intubated endotracheal tube firmly while the fixing device itself is movable along the patient's teeth.

More specifically, this objective may be obtained by adjusting the upper wing 3 after inserting the fixing device into the patient's mouth. It is also possible to provide a fixing device capable of meeting various medical situations with no need that it be custom-made for the patient.

Preferably, the outer surface of the body 1 is spaced from the upper wing 3 at a distance so that the upper wing 3 is movable along the body without exerting too much force. It is also possible to restrict the range of movement of the upper wing 3 by making the top and bottom bosses 18 protrude more than other bosses 18 located between top and bottom ones. Preferably, the upper wing 3 is movable along the outer surface of the body 1 by forcing the upper wing 3 to pass by a boss 18.

Referring to FIG. 18, the upper wing 3 preferably has a portion 31 that corresponds to the boss 18. For example, if the boss 18 is convex, the upper wing 3 has a concave portion 31 so as to correspond to the boss 18. By making the shape of the upper wing 3 correspond to the shape of the boss 18, the upper wing 3 is more easily movable along the boss.

Referring to FIG. 19, the inner surface of the wing 3 is connected to the outer surface of the body 1 where the body 1 is threaded on its outer surface, while the upper wing 3 is correspondingly threaded on its inner surface. The upper wing 3 may be screwed along threads 19a on the outer surface of the body 1 so that the upper wing 3 can progress in the longitudinal direction of the body 1.

Here, the size and shape of the upper wing 3 should correspond to the mouth structure of the patient so that the rotation of the upper wing 3 inside the mouth may not become blocked. Like the boss 18 on the body 1, the threads 19a are formed partially on the outer surface of the body so as to restrict the range of movement of the upper wing 3.

FIG. 20 shows a fixing device according to another embodiment of the present invention, which comprises an airway maintaining element 7 connected to the lower wing 5. The airway maintaining element 7, which is used to prevent the patient's tongue from blocking his airway during surgery, particularly in an emergency situation, may be one with conventional structure. The airway maintaining element 7 not only maintains the airway of the patient more reliably, but it also prevents the patient from consciously or unconsciously pushing out the fixing device using his tongue.

In addition, in an emergency, when it is very important to secure the airway of the patient, the airway maintaining element 7 enables the device of the present invention to secure the patient's airway safely as well as to fix the endotracheal tube, without losing any of the advantages of the present invention.

Since the endotracheal tube is required to pass through the airway maintaining element 7 of the present invention, the airway maintaining element 7 preferably has a hollow portion 73 into which the tubular member 1002 is inserted. The hollow portion 73 preferably is cylindrical with its axis at the center of the airway maintaining element 7 being its axis.

The airway maintaining element 7 may also have one or more wings 71. It is preferable to have two wings 71, each of which is adjacent to the hollow portion 73 so as to broaden the contact area between the wings 71 and the patient's tongue, which must be kept away from his airway.

Referring to FIG. 22, the fixing device of the present invention has a plurality of multipurpose holes 31 and 51. While one or both of the upper and lower wings 3 and 5 may have the multipurpose holes, FIG. 22 shows multipurpose holes 31 and 51 provided on both wings 3 and 5. The multipurpose holes 31 of the upper wing 3 are provided for insertion of an additional member, which helps fix the endotracheal tube more firmly or perform suction more easily. Although the fixing device of the present invention fixes the intubated endotracheal tube even without the multipurpose holes, the multipurpose holes make it possible to provide stronger fixation.

In addition, conventionally, it has been very problematic to perform suction, which is required in surgery on the interior of the mouth or the tonsils, either because an additional suction device has had to be inserted around the intubated endotracheal tube or because it was necessary to unfix the endotracheal tube. The multipurpose holes solve this problem as well.

That is, the additional member, such as a fixing string coupled with the multipurpose holes 31, fixes the endotracheal tube more firmly for performing surgery with the patient in a position, such as a prone position, where there is a greater likelihood for the intubated endotracheal tube to move. For example, the fixing string is inserted into the multipurpose holes 31 and is bound round the neck of the patient after the endotracheal tube is fixed in the insertion hole 11.

It is also necessary for the multipurpose holes 31 to have a diameter big enough for insertion of a suction device. Conventionally, in order to remove a foreign substance from the interior of the mouth or the airway as the surgery demands, the intubated endotracheal tube has had to be unfixed. However, the multipurpose holes 31 of the present invention into which a suction member can be inserted, makes it possible to perform suction without unfixing the intubated endotracheal tube. There is no limit to the number of multipurpose holes although FIG. 22 shows two multipurpose holes 31.

The multipurpose holes 51 of the lower wing 5 may perform the same function as the multipurpose holes 31 of the upper wing 3. However, the multipurpose holes 51 of the lower wing 5 preferably are provided only to support the suctioning because they are not suitable for receiving a fixing string.

Referring to FIG. 23, concave portions 33 and 53 are provided instead of the multipurpose holes 31 and 51. The concave portions 33 and 53 are used to receive an apparatus, such as a bronchoscope, having a diameter too big to be inserted into the multipurpose holes 31 and 51. The concave portions 33 and 53 respectively provided on the upper wing 3 and the lower wing 5, may be biased toward one side of the wings, as the occasion demands.

Referring to FIG. 24, a fixing device according to another embodiment of the present invention comprises a balloon 6 in place of the lower wing 5. The balloon 6 contracts in the course of inserting the fixing device. The balloon 6 plays the same role as the lower wing 5 after being inflated with the air injected by a syringe or the like.

The balloon 6, when inflated, blocks a foreign substance or saliva from entering the airway. It also prevents an anesthetic from dispersing into the air, rather than into the patient's body as needed to provide the desired anesthesia effect.

As the cuff 1003 in FIG. 1 has a tube 1004 and a valve 1005 for its inflation, the balloon 6 may likewise have a tube for inflating it and a valve for injecting air into the tube. The latter tube and valve are not shown in drawings.

Referring to FIG. 25, an insertion groove 12 is provided on the circumferential surface of the body 1, according to another embodiment of the present invention. The insertion groove 12 accommodates the tube 1004, as shown in FIG. 1, for inflating the cuff 1003, while the tube 1004 connects the valve 1005 to the cuff 1003.

If the tubular member 1002 is integrated with the tube 1004, the insertion groove 12 is not necessary. However, when only the tubular member 1002 is inserted into the insertion hole 11 and, then the tube 1004 connects the valve 1005 to the cuff 1003, it is possible to inflate the cuff 1002 easily by inserting the tube 1004 into the insertion groove 12.

Referring to FIG. 26, a fixing device according to another embodiment of the present invention comprises a cap 9. The cap 9 has the shape of a hollow cylinder, has a hole 91 for insertion of the endotracheal tube thereinto, and accommodates the first portion 16a of the body 1.

The diameter of the cap 9 may be smaller than the diameter of the first portion 16a. Here, the first portion 16a can be pushed into the cap 9 by force because the cap 9 is made from deformable elastic materials, such as rubber and polyurethane. The inserted endotracheal tube 1002 can be firmly fixed by the contraction of the insertion hole 11 resulting from contraction of the first portion 16a caused by the pressure applied by the cap 9. Such as elastic material may be applicable to other embodiments of the present invention.

Referring to FIG. 27, the first portion 16a may include cut grooves 14. The first portion 16a is contracted toward the cut grooves 14 by the pressure of the cap, which accommodates the first portion 16a by push-fitting the first portion 16a into the cap 9. Accordingly, the tubular member 1002 is fixed firmly to the first portion 16a. This embodiment does not require the cap material to be elastic, unlike the embodiment according to FIG. 26.

The cut groove 14 is applicable to any embodiments of the present invention and further explanation is omitted in the following embodiments.

FIGs. 28 - 33 show various embodiments of the cap 9. Referring to FIG. 28, the cap 9 has threads 92a on its inner circumferential surface. Here, the outer surface of first portion 16a has threads 161 a corresponding to the threads 92a. By means of those threads, the first portion 16a and the cap 9 can be easily and firmly connected together.

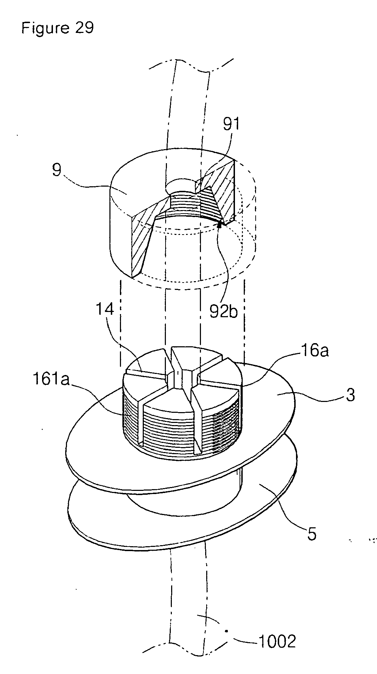

Referring to FIG. 29, the cap 9 may have an inclined surface 92b inside its sidewall. The radius of the cap at a lower portion of the inclined surface 92b is bigger than at a higher portion of the inclined surface so that the connection of the first portion 16a to the cap 9 fixes the inserted endotracheal tube more firmly. The inclined surface. 92 may also have threads thereon so as to fix the intubated endotracheal tube more strongly.

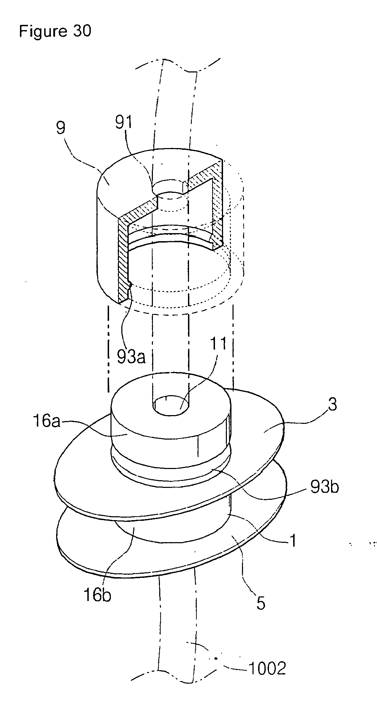

Referring to FIGs. 30 - 32, the cap 9 may comprise respectively a push-fitting boss 93a, a convex portion 94a, or a male latch element 95a. As shown in FIGs. 30 - 32, the first portion 16a coupled with the cap 9 comprises respectively a push-fitting groove 93b, a concave portion 94b, or a female latch element 95b. The push-fitting boss 93a, the convex portion 94a, or the male latch element 95a supports the coupling of the cap 9 with the first portion 16a.

It is also possible for the cap 9 to comprise a push-fitting groove, a concave portion, or a female latch portion, which are not shown, while the first portion 16a would comprise a push-fitting boss, a convex portion, or a male latch portion, also not shown.

The cap 9 and the first portion 16a can be coupled together by the same method as explained in previous embodiments, including the push-fitting between the boss 93a and the groove 93b, the press-button type connection between the convex and concave portions 94a and 94b, and the latch connection between the male and female latch elements 95a and 95b.

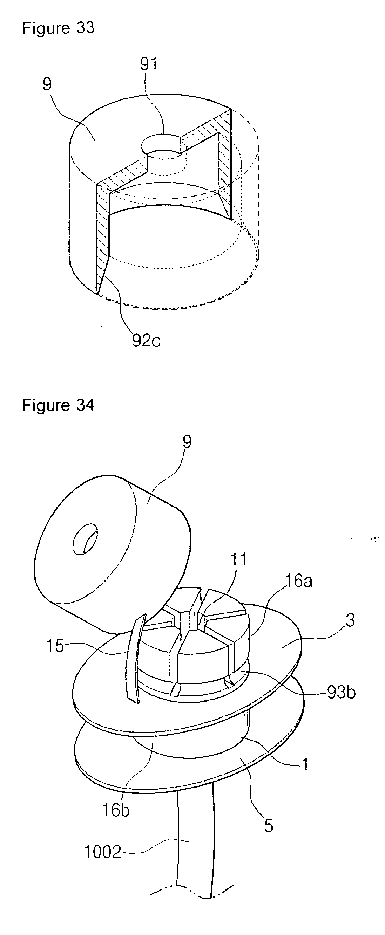

Referring to FIG. 33, the bottom of the cap 9 has a guide surface 92c. The guide surface 92c, which contacts first the edge of the body 1 when the cap 9 is coupled with the body 1, makes it possible to locate the cap 9 with respect to the body 1 exactly and easily.

FIG. 34 shows a fixing device according to another embodiment of the present invention. A portion of the cap 9 is pivotably coupled with a portion of the body 1 through a strap 15, providing various connections between the cap 9 and the first portion 16a, as explained in previous embodiments. This embodiment in which the cap 9 is partially coupled with the body 1 provides a number of advantages, e.g., prevention of the loss of the cap 9.

Referring to FIGs. 35 - 37, the cap 9 is divided into two parts, including a first part 9a and a second part 9b, and the first part 9a respectively has a push-fitting boss 96a, a convex portion 97a, or a male latch element 98a.

Correspondingly, the second part 9b has a push-fitting groove 96b in FIG. 35, a concave portion 97b in FIG. 36, and a female latch element 98b in FIG. 37. An edge of the second part 9b at least partially contacts an edge of the first part 9a. The first and second parts 9a and 9b can be coupled together by the same method as explained in previous embodiments, including the push-fitting between the boss 96a and the groove 96b, the press-button type connection between the convex and concave portions 97a and 97b, and the latch connection between the male and female latch elements 98a and 98b.

In addition to dividing the cap 9 into two parts 9a and 9b and connecting those two parts together by means of a coupling element, the diameter 91 D of the hole 91 of the cap 9, as shown in FIG. 12a, may be smaller than the diameter of the diameter d of the endotracheal tube in order to elastically deform the first portion 16a by force when inserting the endotracheal tube into the hole 91. The cut groove 14 or threads in previous embodiments are applicable to this embodiment.

Referring to FIGs. 38 - 40, the cap 9 is completely divided into the first and second parts 9a and 9b and, then, those two parts are assembled together in the same way as in FIGs. 35 - 37, whose details are omitted.

The diameter of the inner surface of the cap 9, as shown in FIGs. 35 - 40, is preferably smaller than the diameter of the outer surface of the first portion 16a so that the first portion 16a of the body may be push-fitted in the cap 9 by force while the first and second parts 9a and 9b are assembled together. Here, it is also noted that the cap 9 in FIGs. 35 - 40 can be coupled together with the first portion 16a by the same method as explained in previous embodiments, including the push-fitting, the press-button type connection, and the latch connection, and the details are omitted.

Now, the way to use the fixing device is explained according to an embodiment of the present invention. Among various embodiments of the present invention, the way to use the fixing devices would be explained by taking the fixing device having the cap 9 as an example, referring to FIGs. 41 and 43. However, it is noted that the scope of the present invention is not limited to such an example.

First, the body 1 is inserted into the patient's mouth in an emergency when surgery inside mouth is necessary. When inserting the body 1, the bottom of the upper wing 3 should contact the front surface of the patient's teeth while the top of the lower wing 5 contacts the rear surface of the teeth by adjusting the length of the second portion 16b so that the second portion is appropriately engaged with the teeth. Next, after determining the exact inserting depth of the endotracheal tube by using a laryngoscope or the like, the tubular member 1002 is inserted into the hole 91 of the cap 9 and the insertion hole 11 of the body 1.

When the endotracheal tube is inserted to the exact depth, e.g., the depth where the cuff protrudes 1~2 cm from the glottis, the endotracheal tube is temporarily fixed by inflating the cuff 1003 with air which is injected by the syringe via the cuff-inflating valve 1005. Next, the cap 9 and the first portion 16a are coupled together, for example, by rotating the cap 9 along the threads on the body 1. In addition, a stronger fixation is possible when the cap 9 has the incline surface 92b, the first portion 16b is made from elastic material, or the first portion has the cut groove 14b. This is because the first portion 16a contracts as the screw type connection proceeds, so that the insertion hole 11 also contracts and thus fixes the circumferential outer surface of the endotracheal tube firmly.

Accordingly, when the screw type connection between the cap 9 and the body 1 is completed, the tubular member 1002 is firmly fixed to the body 1 so that the lower wing 5 contacts the rear surface of the teeth T while the upper wing 3 contacts the front surface of the teeth T. In this way, the body 1 of the fixing device is prevented from moving backward and forward, for example, from moving in the direction C as shown in FIG. 42 while it maintains the location of the inserted endotracheal tube even when the patient consciously or unconsciously tries to extract the inserted endotracheal tube. Accordingly, the endotracheal tube is fixed firmly and the cuff 1003 may be inflated exactly to the extent that it prevents the oxygen or the anesthetic from flowing backward, and a sore throat resulting from overinflation of the cuff can be avoided.

As aforementioned, the fixing device of the present invention can move from side to side, for example in the direction B as shown in FIG. 41. Thus, the fixing device of the present invention secures a broader space for performing surgery by dentists, plastic surgeons or otorhinolaryngologists and enables easier suction during such surgery.

In addition, the fixing device provided with the airway maintaining element 7, as shown in FIG. 43, broadens the contact area between the wings 71 and the patient's tongue and, thus, prevents rolling of the tongue and, accordingly, prevents the endotracheal tube from changing its location.

As is apparent from the above description, the present invention provides a fixing device that can fix the endotracheal tube more firmly by means of the cap and the body, rather than a cuff and an adhesive bandage, so that it makes it less likely that the inserted endotracheal tube may move and come out from the fixed location even when the patient consciously or unconsciously touches the inserted endotracheal tube.

In addition, the present invention provides a device which is capable of fixing an endotracheal tube without using an adhesive bandage, so that it prevents contact dermatitis. The present invention also enables a single person to fix the endotracheal tube easily and exactly, because the endotracheal tube easily can be inserted into the hole of the cap and the insertion hole of the body during intubation, and easily can be fixed with the body by rotating the cap, so that labor costs are reduced and emergencies can be met more efficiently.

Further, the present invention provides a device that is capable of fixing an intubated endotracheal tube more firmly without overinflating the cuff, and maintaining the endotracheal tube exactly at the fixed, position when the tube is used in surgery on the interior of the mouth or in surgery on a patient in a prone position.

Furthermore, the present invention provides a device that is capable of fixing an endotracheal tube more firmly by means of a multipurpose hole accommodating an additional fixing member and providing simpler and easier suction when suction is necessary.

Further, the present invention provides a device movable along a patient's teeth, so that the inserted endotracheal tube can be moved without extracting and re-fixing the endotracheal tube.

Further, the present invention provides an endotracheal tube fixing device that maintains an airway of a patient without providing an additional device specifically for maintaining the airway.

Further, the present invention provides an endotracheal tube fixing device, which fixes the endotracheal tube accurately and safely and, accordingly, prevents a complication such as a sore throat by preventing an overinflation of a cuff.

Although several embodiments of the present invention have been shown and described, it will be appreciated by those skilled in the art that changes may be made in these embodiments without departing from the principles and spirit of the invention, the scope of which is defined in the appended claims and by their equivalents.

## Claims

1. A device for fixing an endotracheal tube comprising:
a body having an insertion hole;
an upper wing on a periphery of the body; and
a lower wing on the periphery of the body, the lower wing being spaced from the upper wing such that the teeth of a patient are accommodated between the lower wing and the upper wing.

2. The device of claim 1, further comprising a cap and wherein:
the cap has a concave portion; and
the concave portion accommodates a portion of the body so as to fix the endotracheal tube after the endotracheal tube is inserted into the insertion hole.

3. The device of claim 2, wherein:
the body comprises a first portion protruding from the first wing;
and the first portion has a cut groove.

4. The device of claim 3, wherein the cap has an inclined sidewall so that the radius of the cap at its lower portion is bigger than at its higher portion.

5. The device of claim 2, wherein the cap has a thread on an inner surface of its sidewall and the body has a thread on its outer surface.

6. The device of claim 5, wherein the inner surface of the sidewall is inclined so that the radius of the cap at a lower portion of the inner surface is bigger than at a higher portion of the inner surface.

7. The device of claim 2, wherein the body comprises a push-fitting groove on an outer surface thereof and the cap has a push-fitting boss corresponding the push-fitting groove on an inner surface thereof.

8. The device of claim 7, wherein the push-fitting boss is bigger than the push-fitting groove.

9. The device of claim 7, wherein the body has a cut groove.

10. The device of claim 2, wherein the body has a concave portion on an outer surface thereof and the cap has a convex portion on an inner surface thereof.

11. The device of claim 10, wherein the body has a cut groove.

12. The device of claim 2, wherein the body has a female latch element on an outer surface thereof and the cap has a male latch element on an inner surface thereof.

13. The device of claim 12, wherein the body has a cut groove.

14. The device of claim 2, wherein:
the cap has a first part and a second part; and
the first part and the second part are connected together and a portion of the body is accommodated therein so as to fix the endotracheal tube.

15. The device of claim 14, wherein the body has a cut groove.

16. The device of claim 14, wherein the first part has a push-fitting boss and the second part has a push-fitting groove.

17. The device of claim 16, wherein the push-fitting boss is bigger than the push-fitting groove.

18. The device of claim 14, wherein the first part has a concave portion and the second part has a convex portion.

19. The device of claim 14, wherein the first part has a male latch element and the second part has a female latch element.

20. The device of claim 2, wherein the cap comprises a guide surface at a bottom thereof.

21. The device of claim 2, wherein the cap has a sidewall whose inner surface is inclined.

22. The device of claim 2, wherein the cap is partially coupled with the body.

23. The device of claim 1, further comprising a supplementary insertion element that is inserted into the insertion hole after the endotracheal tube is inserted into the insertion hole.

24. The device of claim 1, further comprising a bonding member and wherein:
the bonding member bonds the body and the endotracheal tube together after the endotracheal tube is inserted into the insertion hole.

25. The device of claim 1, wherein:
the body comprises a first body and a second body; and
the first body and the second body are connected together so as to fix the endotracheal tube after the endotracheal tube is inserted into the insertion hole.

26. The device of claim 25, wherein the first body comprises a push-fitting boss and the second body comprises a push-fitting groove.

27. The device of claim 26, wherein the push-fitting boss is larger than the push-fitting groove.

28. The device of claim 25, wherein the first body comprises a concave portion and the second body comprises a convex portion.

29. The device of claim 25, wherein the first body comprises a male latch element and the second body comprises a female latch element.

30. The device of claim 1, wherein:
the upper wing is spaced apart from the body;
the body has bosses on an outer surface thereof; and
the upper wing is movable along the outer surface of the body so as to be usable on patients with various shapes of the teeth and the interior of the mouth.

31. The device of claim 1, wherein:
the body has a thread on an outer surface thereof;
the upper wing has a thread on an inner surface thereof;
the upper wing is slidable and fixable along the outer surface of the body.

32. The device of claim 1, further comprising an airway maintaining element that is connected to the lower wing.

33. The device of claim 32, wherein the airway maintaining element comprises:
a hollow portion into which the endotracheal tube is inserted; and
a wing adjacent to the side of the hollow portion.

34. The device of claim 1, wherein the upper wing has a plurality of multipurpose holes.

35. The device of claim 34, wherein the lower wing has a plurality of multipurpose holes.

36. The device of claim 1, wherein:
the lower wing is balloon-shaped;
the lower wing is contracted in the course of inserting and extracting the device; and
the lower wing inflates while the device fixes the endotracheal tube.

37. The device of claim 36, wherein the upper wing has a plurality of multipurpose holes.

38. The device of claim 1, wherein the upper and lower wings each have a concave portion.

39. The device of claim 38, wherein the concave portion is circular or oval.

40. The device of claim 1, wherein the body has an insertion groove for fitting therein a tube that is used to inflate a cuff of the endotracheal tube.

41. The device of claim 40, wherein the insertion groove is smaller in diameter than the tube.
